# EUROPEAN PATENT APPLICATION

(11) **EP 1 739 605 A1**
(43) Date of publication of application: **03.01.2007**
(21) Application number: 05730544.3
(22) Date of filing: 13.04.2005
(51) Int. Cl.: G06Q 10/00

(54) **CLINICAL COMMUNICATION UNIT AND HOSPITAL INFORMATION SYSTEM**

(30) Priority: 14.04.2004 JP 2004118903
(71) Applicant: Koninklijke Philips Electronics N.V., 5621 BA Eindhoven (NL)
(72) Inventor: KOMIYA, Masayoshi c/o Philips Electronics Japan,, Tokyo 1088507 (JP); TANAKA, Mitsuhiko; c/o Daishin Giken Co.,, 8593223 (JP)
(74) Representative: Volmer, Georg
(86) International application number: PCT/JP2005/007160
(87) International publication number: WO 2005/101262

(57) **Abstract**

Provided is a clinical communication device and a hospital information system that are capable of information exchange while linking electronic medical records with event information for patients and doctors. An electronic medical record information extraction section (13) extracts electronic medical record information from an electronic medical record database (3) and sends it to an information update section (15) and/or a display control section (16). A clinical communicator information extraction section (14) extracts clinical communicator information from a clinical communicator database (4) and sends it to the information update section (15) and/or the display control section (16). The display control section (16) displays the electronic medical information and the clinical communicator information, while linking them with each other.

## Description

### TECHNICAL FIELD

The present invention relates to clinical communication devices and hospital information systems, and particularly to a clinical communication device and a hospital information system that are capable of information exchange, while linking electronic medical records with event information for patients and doctors.

### BACKGROUND ART

Recently, there are an increasing number of hospitals in which an electronic medical record system has been introduced or which are considering introduction of the system. The electronic medical record system is an information management system that handles various types of clinical information, including specimen examination results, imaging diagnosis information, nursing records as well as diagnosis and treatment records for doctors. Among institutions where the electronic medical record system has been introduced, there are more than a few such institutions where commercially available groupware has been introduced in order to manage groupware and promote communications between medical care professionals such as doctors and nurses.

In addition, among institutions where an information management system such as the electronic medical record system has been introduced, there are an increasing number that have installed therein process control tools such as clinical pathways. The clinical pathway is an application of a critical pathway, which is an industrial manufacturing process schedule, to medical care, and is alternatively referred to as "care map" or "care pathway". The clinical pathway is "organized in the form of a table illustrating the details of prerequisite (critical) work that has to be done by a medical team in order to achieve a specific result (outcome) for a patient having a specific disease, and the most desirable order or time of implementation". This is a notion of applying industrial process control, such as operational control, time control and cost control, to medical care, thereby conceivably improving efficiency to ensure successful performance and reduce days of hospital stay and cost.

The clinical pathway, which is introduced in a number of institutions, is in the form of matrix with the vertical axis showing scheduled implementation items and the horizontal axis showing dates, in which scheduled items to be implemented during days since hospitalization until discharge are shown. Implementation schedule, which shows which medical care is to be carried out on which day after hospitalization or what has to be completed on which day before the date of surgery, is set based on previous achievements and an objective. The clinical pathway is relatively easily standardized into a pathway for femur replacement or CABG (coronary artery bypass graft), and is applied to the case where unexpected events are unlikely to take place and there are a number of cases, but recently, a number of hospitals for acute-stage illness are demanded to further reduce the average number of days of hospital stay, so that there are an increasing number of cases where the application of the process control tool such as the clinical pathway is expanded.

### DISCLOSURE OF THE INVENTION

### PROBLEM TO BE SOLVED BY THE INVENTION

The clinical pathway, which is currently used in a number of institutions, is, however, a standard plan and does not have a rescheduling or coordination function for, if once an unscheduled event has taken place, rendering the process optimal and shortest in the current situation. For example, consider a case where there is a clinical pathway in which a final decision for discharge is made after checking the result for imaging examination such as CT, but CT examination cannot be carried out because the condition of the patient has changed suddenly. Although it is possible to automatically extend the pathway by one day or reschedule the examination to an arbitrary date, the rescheduling is not carried out after checking availability of CT examination and subsequent plans for the doctor and other medical care professionals, and therefore optimal rescheduling cannot be actually carried out.

In addition, commercially-available groupware is operated separate from the electronic medical record system, and does not share information therewith, and therefore it is required to reenter necessary information by cutting and pasting the content of the clinical pathway on the electronic medical record system, which is considerably troublesome.

The present invention has been made in consideration of the above, and aims to provide a clinical communication device and a hospital information system that are capable of information exchange, while linking electronic medical records with event information for patients and doctors.

### MEANS FOR SOLVING PROBLEM

A clinical communication device of the present invention includes: electronic medical record information extraction means for extracting electronic medical record information from an electronic medical record database, the electronic medical record information including at least clinical pathways, medical record information for patients and follow-up sheets for the patients; clinical communicator information extraction means for extracting clinical communication information from a clinical communicator database, the clinical communication information including at least event information for the patients, event information for medical care professionals and messages to/from the medical care professionals; information update means for updating the electronic medical record information and/or the clinical communication information in accordance with an input; and display control means for displaying the electronic medical record information and the clinical communication information, while linking them with each other.

With this structure, the electronic medical record information and the clinical communication information are displayed, while linking them with each other, and therefore communications between the medical care professionals are promoted, whereby it is possible to accurately and quickly address current conditions of the patients and set optimum pathways for the patients. Thus, it is possible to provide high-quality medical care.

In the clinical communication device of the present invention, it is preferred that the electronic medical record information includes diagnosis information and order information, and the display control means generates a scheduling table containing the event information for the patients and/or the event information for the medical care professionals and displays the diagnosis and treatment records, the order information, the follow-up sheets and/or the clinical pathways from the scheduling table.

In the clinical communication device of the present invention, the information update means preferably sets an importance level for information that is to be exchanged between the medical care professionals.

In the clinical communication device of the present invention, the display control means preferably issues an alert when a scheduled time to implement an event for the patients and/or an event for the medical care professionals has passed.

A hospital information system of the present invention includes: an electronic medical record database having stored therein electronic medical record information including at least clinical pathways, medical record information for patients and follow-up sheets for the patients; a clinical communicator database having stored therein clinical communication information including at least event information for the patients, event information for medical care professionals and messages to/from the medical care professionals; and the clinical communication device connected to the electronic medical record database and the clinical communicator database through a communication link.

With this structure, schedule management can be carried out with reference to events for medical care professionals and in-hospital events or information provided from the medical care professionals, and therefore it is possible to perform reality-based process control.

### EFFECT OF THE INVENTION

According to the present invention, it is possible to display the electronic medical record information and/or the clinical communication information, while linking them with each other, and therefore it is possible to share necessary information mainly related to patients among medical care professionals including doctors, nurses, pharmacists, therapists and other medical care professionals. In addition, according to the present invention, it is possible to perform communications between various occupational positions, promoting group practice. Further, according to the present invention, it is possible to collectively display main events scheduled in a patient's care plan, schedules of individual medical care professionals such as doctors and nurses and events scheduled in the hospital, making it possible to design a more realistic care plan.

### BEST MODE FOR CARRYING OUT THE INVENTION

Process control tools called "clinical pathways" have been introduced for optimization, but the current clinical pathways have a significant functional problem, and therefore are actually used by few institutions. The present inventors started to study the process control tools. However, the problem cannot be solved by a single function of clinical pathway, and close combination with another function is prerequisite. It takes a great deal of cost to realize such technology, and realizing template information, etc., which provides a basis therefor, presents significant difficulties, which are too great to be addressed now.

Therefore, the present inventors slightly changed their viewpoint from the angle of process control, and devised a tool for promoting group practice. Thus, they arrived at a clinical communication device (a clinical communicator) according to the present invention. Specifically, the present inventors integrated a clinical application with groupware, and found a new function of exchanging information while linking electronic medical records with event information for patients and doctors.

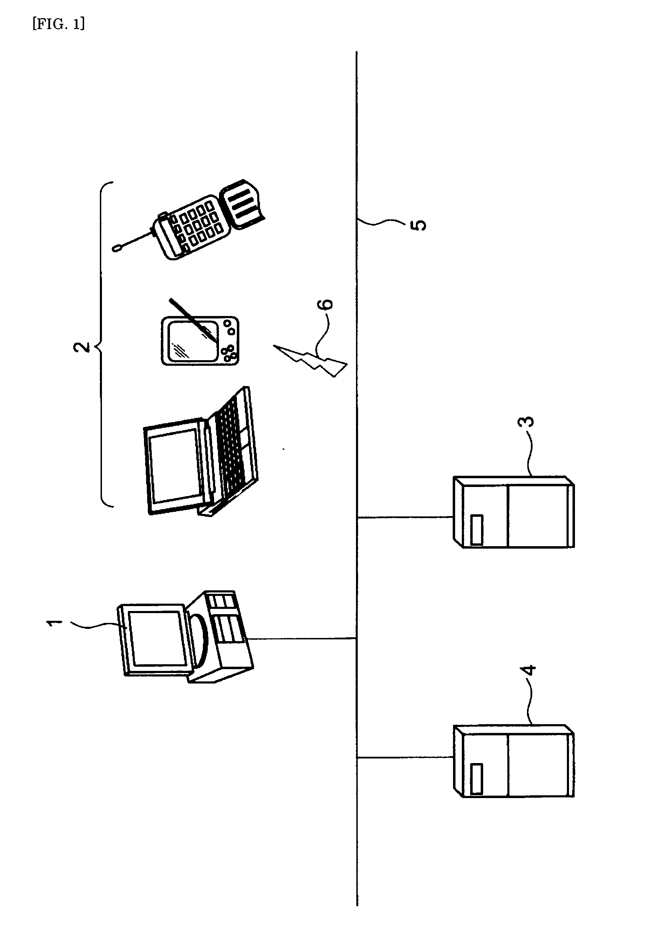

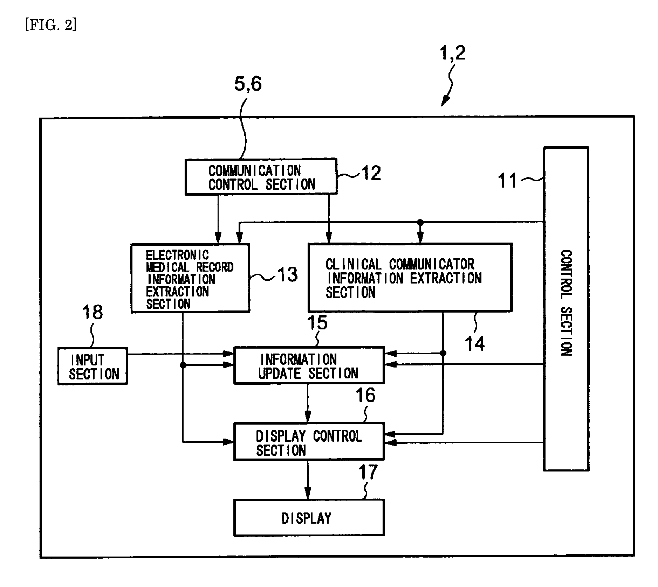

Hereinafter, a clinical communication device and a hospital information system according to an embodiment of the present invention will be described with reference to the attached drawings. FIG. 1 is a diagram illustrating a schematic configuration of a hospital information system according to an embodiment of the present invention. FIG. 2 is a diagram illustrating a schematic configuration of a clinical communication device according to an embodiment of the present invention.

The hospital information system shown in FIG. 1 includes a clinical communication device 1 according to the present invention, an electronic medical record database 3 having stored therein electronic medical record information containing at least clinical pathway information, medical record information for patients and follow-up sheets for the patients, and a clinical communicator database 4 for clinical communication information containing at least event information for the patients, event information for medical care professionals and messages to/from the medical care professionals. The clinical communication devices 1 and 2 are connected with the electronic medical record database 3 and/or the clinical communicator database 4 via a communication link 5. The communication link 5 includes a network such as a LAN or the Internet.

The clinical communication devices 1 and 2 may be desktop personal computers or notebook personal computers, or may be mobile devices such as PDAs (Personal Digital Assistants) or cell phones. In the case where the clinical communication device 2 is a mobile device, the communication link is a radio link 6. In addition, in the case where a mobile device is used as the clinical communication device, the electronic medical record information and the clinical communicator information may be only browsable.

Although FIG. 1 illustrates a case where the electronic medical record database 3 and the clinical communicator database 4 are separately provided, the present invention may be configured such that the electronic medical record database 3 and the clinical communicator database 4 are managed in the same database.

As shown in FIG. 2, the clinical communication device mainly includes a control section 11 for controlling the entire device, a communication control section 12 for sending/receiving necessary information via the communication link 5 or 6, an electronic medical record information extraction section 13 for extracting the electronic medical record information from the electronic medical record database 3, a clinical communicator information extraction section 14 for extracting the clinical communicator information from the clinical communicator database 4, an information update section 15 for updating the electronic medical record information and/or the clinical communicator information in accordance with an input from an input section 18, and a display control section 16 for performing display control when displaying the electronic medical record information and/or the clinical communicator information on a display 17.

The electronic medical record database 3 has stored therein electronic medical record information including at least clinical pathways (care plans), medical record information for patients, and follow-up sheets for patients. Examples of clinical pathway-related information include plans for implementing treatment such as injection, prescription, examination and care. The clinical pathway-related information is set for each patient with the purpose of treatment standardization and cost reduction except in complicated cases or in the case of urgent hospitalization, for example.

The medical record information for patients is information concerning doctors' diagnosis and treatment record sheets in Form No. 2. The medical record information for patients is, for example, diagnosis information concerning diagnosis and treatment records, etc., order information concerning injection, prescription, examination, etc., performed at the time of medical examination, and previous histories thereof. In addition, information concerning a follow-up sheet (a flow sheet) for a patient is, for example, information concerning vital signs (heart rate, blood pressure, body temperature, respiration rate, etc.), In/Out information (transfusion, urine flow, hemorrhage, etc.), observation information (state of consciousness, skin condition, etc.), a care implementation result, an injection/prescription implementation result, or an examination result (blood gas, biochemical, etc.). These pieces of information are preferably displayed in the form by which the tendency of progress is known.

In addition, other types of electronic medical record information include, for example, nursing information, a progress record (in which problems/measures during nursing, results, etc., are organized in the form of SOAP (Subject, Object, Assessment, Plan) or DAR (Data, Action, Response)), and image information (results for imaging examination based on MR, CT, x-rays or the like).

The clinical communicator database 4 has stored therein clinical communication information including at least event information for patients, event information for medical care professionals, and messages to/from the medical care professionals. The event information for patients is information that is included in plan information set by a clinical pathway and concerns events having a high importance level in a care plan, e.g., surgery, pre-surgery examination, an expected date of weaning and an expected date of discharge.

The event information for medical care professionals is, for example, information indicating that a doctor is absent because of an academic conference or he/she is attending an in-hospital meeting. The messages to/from the medical care professionals are, for example, pieces of information conveying patients' conditions from a nurse to a doctor or from a doctor to a nurse, a caregiver or a therapist. The messages are preferably sent and received in the form of email. The information can be readily exchanged between the medical care professionals through email. In addition, it is preferred to have a reminder function for notifying of newly arrived mail and unchecked mail.

In addition, other types of clinical communication information include indices for invoking electronic medical record information stored in an electronic medical record server, e.g., patient information (ID, name, date to be displayed, etc.).

The communication control section 12 in FIG. 2 controls sending and receiving of the electronic medical record information and/or the clinical communicator information among the clinical communication devices 1 and 2 and the electronic medical record database 3 and/or the clinical communicator database 4 in FIG. 1.

The electronic medical record information extraction section 13 extracts electronic medical record information from the electronic medical record database 3, and sends it to the information update section 15 and/or the display control section 16. The clinical communicator information extraction section 14 extracts clinical communicator information from the clinical communicator database 4, and sends it to the information update section 15 and/or the display control section 16.

The information update section 15 updates electronic medical record information, which is inputted through the input section 18, for the electronic medical record database 3, and updates the clinical communicator information for the clinical communicator database 4. The updated information is sent through the communication control section 12 to the electronic medical record database 3 or the clinical communicator database 4 via the communication link 5 or 6.

In addition, the information update section 15 sets, as necessary, an importance level for information that is to be exchanged between medical care professionals, and displays whether the medical care professionals have checked the information (checked or unchecked). By having such a function, it is possible to readily recognize whether the medical care professionals have checked the importance level of the information or the information itself.

The display control section 16 displays the electronic medical record information and the clinical communication information, while linking them with each other. For example, the display control section 16 generates a scheduling table based on event information for a patient and/or event information for medical care professionals. The display control section 16 then displays diagnosis and treatment records, order information, a follow-up sheet and/or a clinical pathway from the scheduling table. As such, events for the patient and in-hospital events can be displayed together with schedules for the medical care professionals, i.e., the electronic medical record information and the follow-up sheet can be displayed from the scheduling table, and therefore the electronic medical record information and the clinical communication information can be mutually exchanged between them.

In addition, the display control section 16 issues an alert as necessary when the scheduled time to implement a patient's event and/or a medical care professional's event has been passed. This allows the medical care professional to be notified of implementation or suspension of the event. Note that the alert may be provided by displaying a colored screen or enlarged characters or by sounding an alarm.

As such, in a hospital information system using the clinical communication device according to the present embodiment, communications between medical care professionals are promoted, whereby it is possible to accurately and quickly address current conditions of patients and set optimum pathways for the patients. Thus, it is possible to provide high-quality medical care. In addition, schedule management can be carried out with reference to events for medical care professionals and in-hospital events or information provided from the medical care professionals, and therefore it is possible to perform reality-based process control. Further, by invoking from the scheduling table other functions such as diagnosis and treatment records, ordering, progress records, clinical pathways, nursing plans and a roster and other systems such as a reservation system, it is possible to smoothly access necessary information without disturbing the thought process, thereby supporting work efficiency.

In addition, the hospital information system will eventually make it possible to promote smooth communications with patients and their families, and will also make it possible to realize home care, community liaison and group practice involving patients.

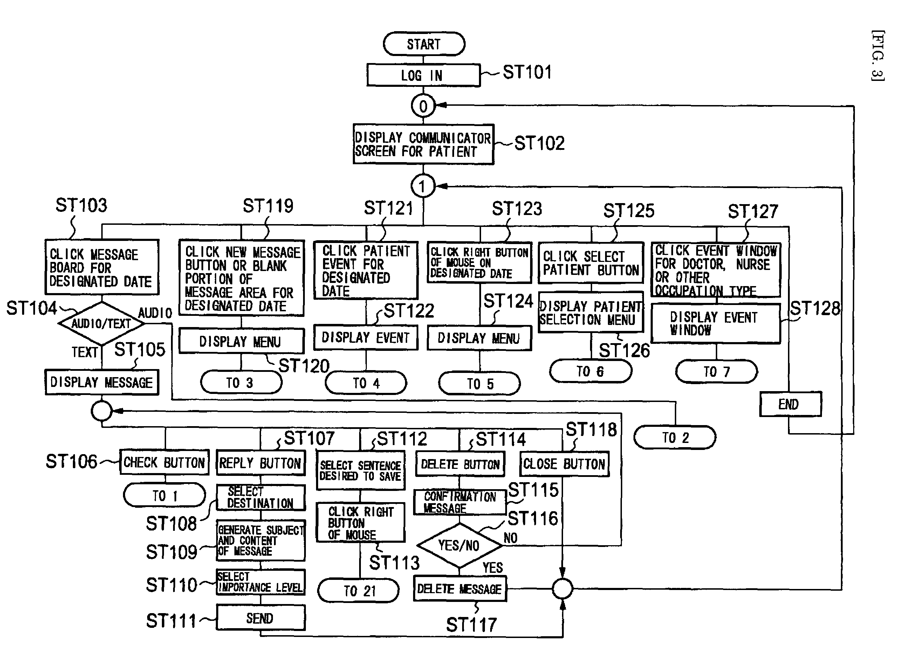

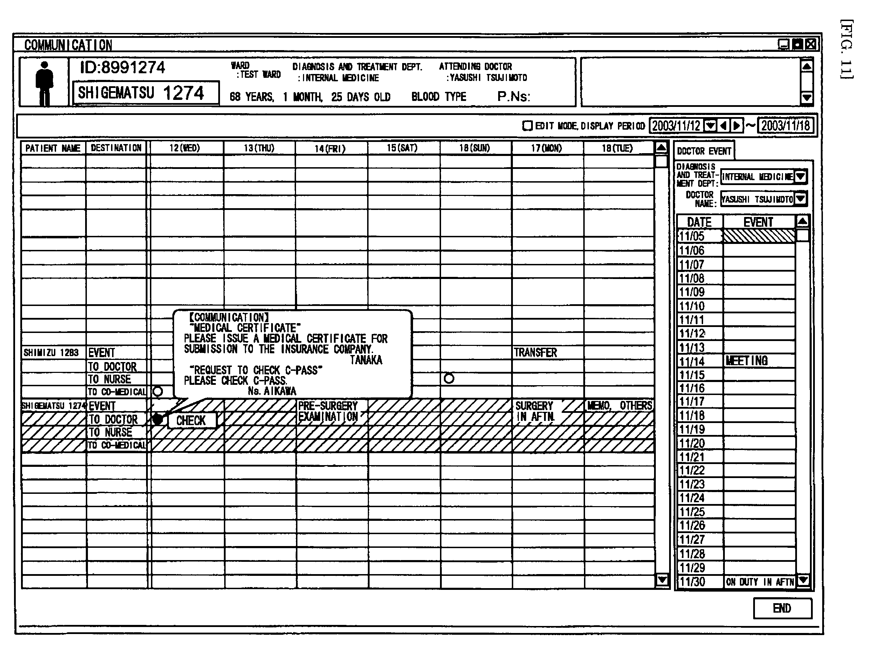

Described next are actual operating procedures of a clinical communication device as configured above. FIGS. 3 through 10 are flow charts for explaining the procedures for operating a clinical communication device according to an embodiment of the present invention. FIGS. 11 through 15 are diagrams each illustrating a display screen when operating the clinical communication device according to the embodiment of the present invention.

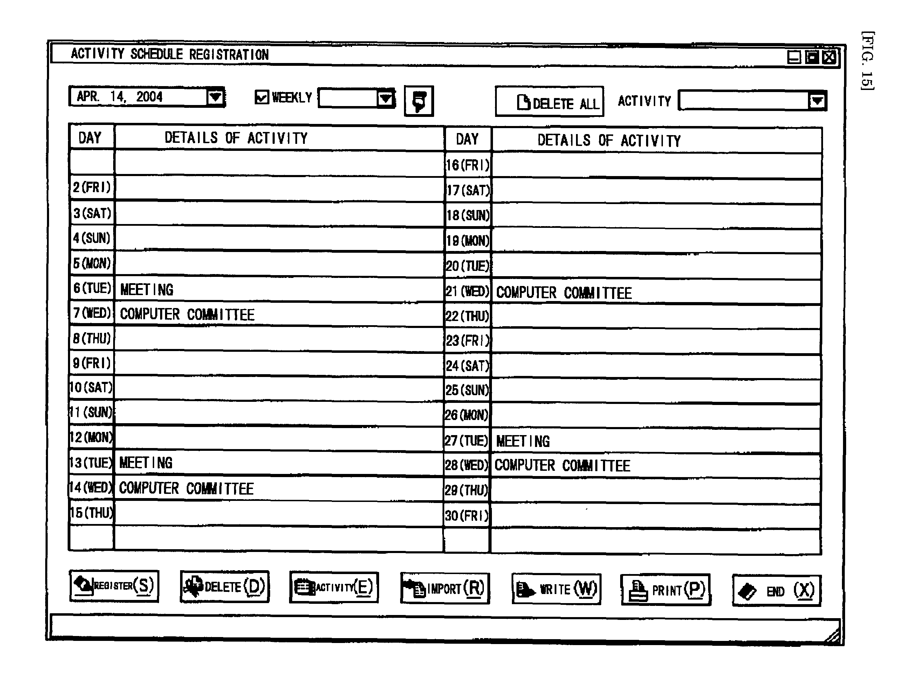

In FIG. 3, when login-in is performed with the clinical communication device (ST101), a communicator screen (a calendar) for a patient assigned to the doctor who has logged in is displayed (ST102) as shown in FIG. 11. In the calendar, an open circle and a filled circle indicate that there are messages sent from a doctor, a nurse, a pharmacist, etc. In the screen shown in FIG. 11, events are displayed in a scheduling table, and message marks are displayed. In addition, displayed on the right side is a scheduling table in which only a personal schedule of the doctor who has logged in and in-hospital events are displayed as shown in FIG. 15. By displaying such a scheduling table in a lower part of a scheduling table, it is possible to make comparison with main events for the patient. Note that in the scheduling table, the main events are displayed in order of priority. In addition, the scheduling table can be selectively displayed either on a week-by-week basis or on a month-by-month basis.

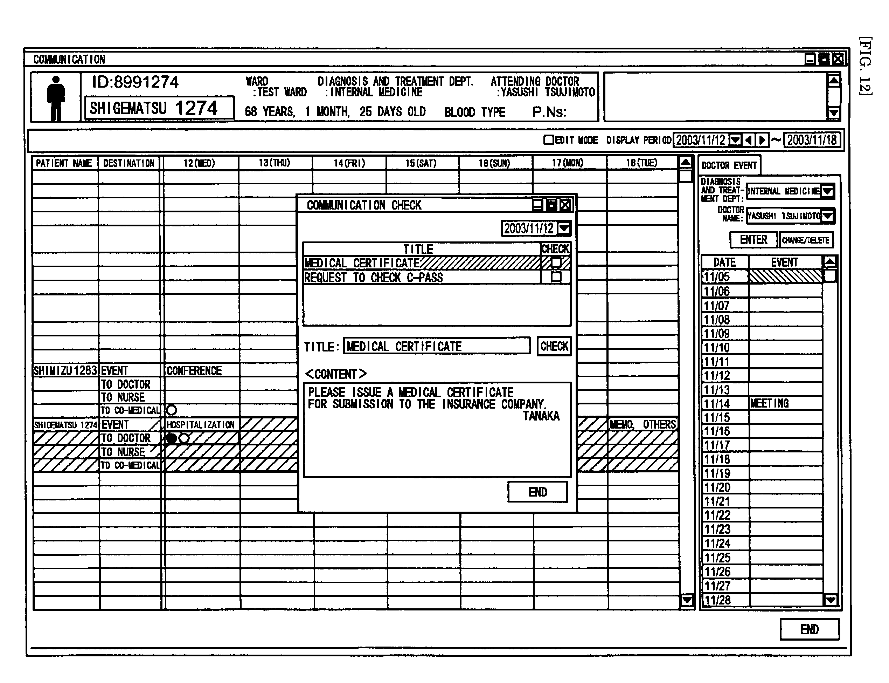

Display colors and markers are differentiated in accordance with importance levels of messages. In addition, whether the content of a message has been checked or unchecked can be recognized by the display color and form of a marker. In the case where there is an unchecked message, the screen indicates the presence of the unchecked message as shown in FIG. 11. By clicking CHECK button, a message check screen is displayed as shown in FIG. 12. Note that messages exchanged between medical care professionals can be copied as necessary onto a diagnosis and treatment record or a nursing record by cut and paste.

After clicking a message box for a designated date (ST103), select whether the message should be audio or text (ST104). If text is selected, the message is displayed on the screen (ST105). If the message is a desired one, then press CHECK button (ST106).

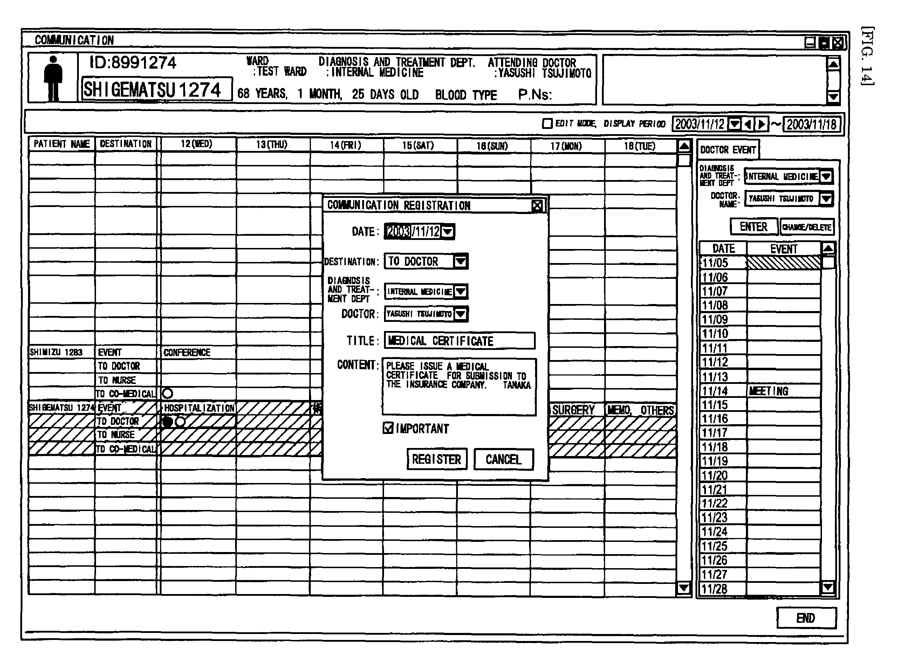

A reply to the message can be made via email. When making a reply, press REPLY button on the screen (ST107), and select a destination (ST108). Then, generate the subject and content of the message (ST109). Thereafter, select the importance level of the message (ST110) before sending the message as shown in FIG. 14 (ST111). As such, the message is sent via email.

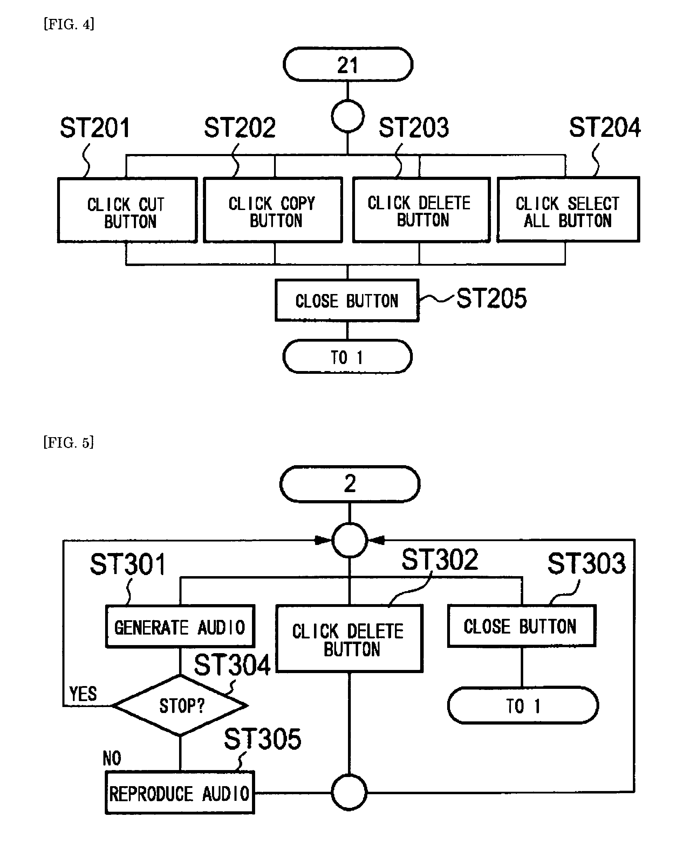

If there is any sentence desired to save, select the sentence (ST112), and click right button of mouse (ST113). In this case, click CUT button (ST201), COPY button (ST202), DELETE button (ST203) or SELECT ALL button (ST204) as shown in FIG. 4. Click CLOSE button after clicking any of the buttons (ST205).

In the case of deleting the message, click DELETE button on the screen (ST114). In this case, when a confirmation message is displayed on the screen (ST115), select "YES/NO" (ST116). If "YES" is selected, then the message is deleted (ST117). When the operation on the message display screen is completed, click CLOSE button (ST118).

In the case where an audio message is selected, reproduce audio (ST301) as shown in FIG. 5; determine whether to press STOP button during the audio reproduction (ST304), and, if the audio reproduction is not to be stopped, continue the audio reproduction (ST305). In the case of deleting audio, click DELETE button (ST302). In addition, in the case of stopping the audio reproduction, click CLOSE button (ST303).

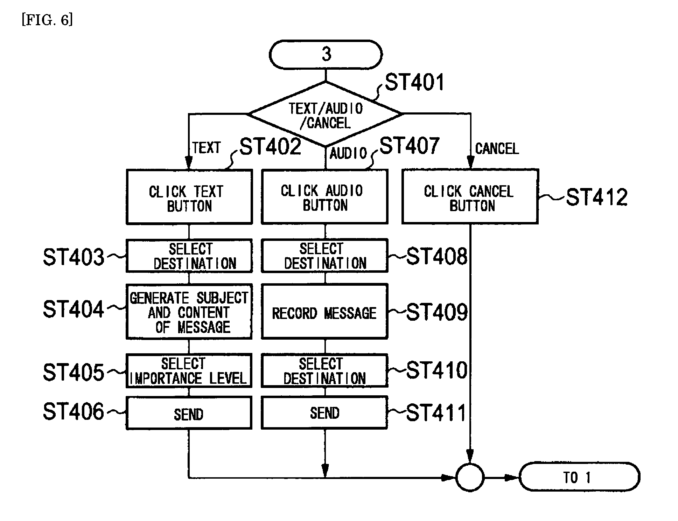

Referring back to FIG. 3, if NEW MESSAGE button or a blank portion of a message area for a designated date is clicked (ST119), a menu screen is displayed (ST120). In the menu screen, select either "TEXT/AUDIO/CANCEL" (ST401) as shown in FIG. 6. In the case of selecting TEXT, click TEXT button (ST402), select a destination (ST403), generate the subject and content of the message (ST404), and select an importance level before sending (ST405, ST406).

In the case of selecting audio, click AUDIO button (ST407), select a destination (ST408), record a message (ST409), and select an importance level before sending (ST410, ST411). In the case of making cancellation, click CANCEL button (ST412).

Referring back to FIG. 3, if a patient event for a designated date is clicked (ST121), an event screen is displayed (ST122). In the event screen, select either "UNLINK PLAN/LINK PLAN/CLOSE" (ST501) as shown in FIG. 7. In the case of selecting UNLINK, check the details of the event (ST502). In this case, select "MODIFY/DELETE/CLOSE" in an event detail screen (ST504). In the case of selecting MODIFY, modify the details (ST505), and save the details of modification in the device (ST506) as shown in FIG. 13. In the case of selecting DELETE, click DELETE EVENT button (ST507). In the case of ending the operation, click CLOSE button (ST510). On the other hand, in the case of selecting LINK, check the details of the event (ST503), and click CLOSE button to end the operation (ST510).

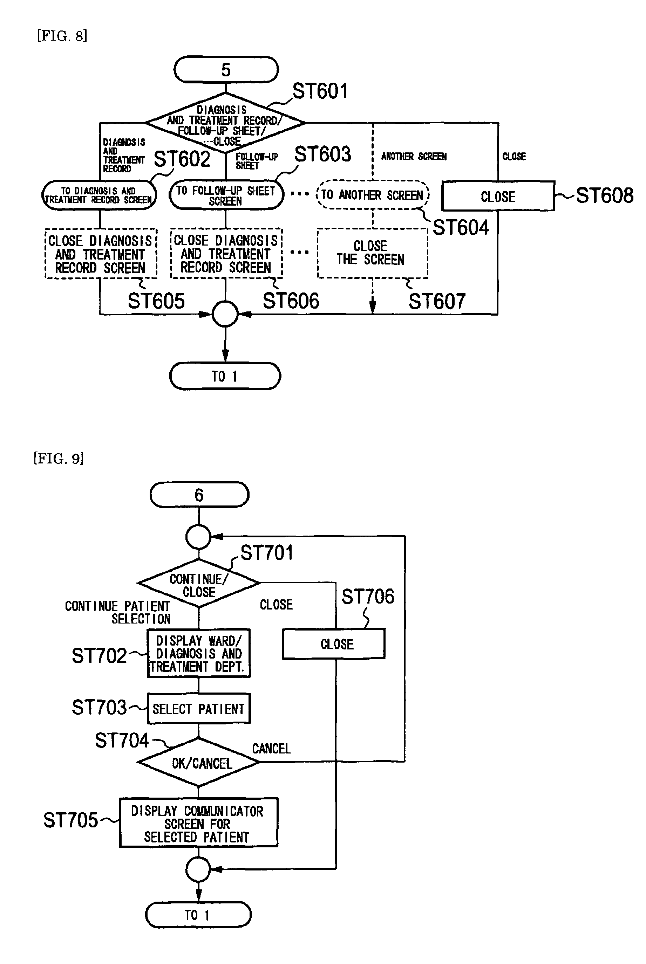

Referring back to FIG. 3, when the right button of the mouse is clicked on a designated date (ST123), a menu screen is displayed (ST124). In the menu screen, select "DIAGNOSIS AND TREATMENT RECORD/FOLLOW-UP SHEET/.../CLOSE" (ST601) as shown in FIG. 8. If DIAGNOSIS AND TREATMENT RECORD is selected, a diagnosis and treatment record screen is displayed (ST602) to operate the diagnosis and treatment record screen. After the operation on the diagnosis and treatment record screen is completed, close the diagnosis and treatment record screen (ST603). If FOLLOW-UP SHEET is selected, a follow-up sheet screen is displayed (ST603) to operate the follow-up sheet screen. After the operation on the follow-up sheet screen is completed, close the follow-up sheet screen (ST606). If another screen is selected, the selected screen is displayed (ST604) to operate that screen. After the operation on the screen is completed, close the screen (ST607). In the case of ending the operation, click CLOSE button (ST608).

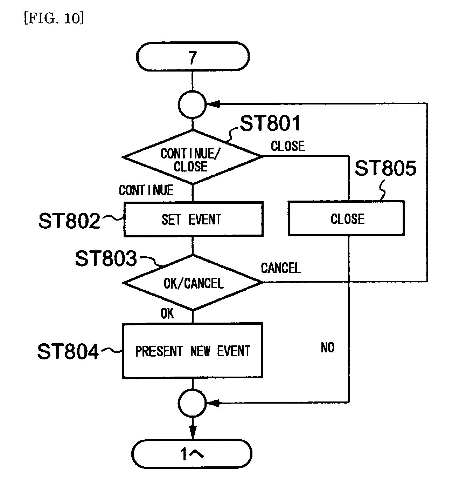

Referring back to FIG. 3, if SELECT PATIENT button is clicked (ST125), a patient selection menu screen is displayed (ST126). In the patient selection menu screen, select "CONTINUE/CLOSE" (ST701) as shown in FIG. 9. If the patient selection is continued, WARD/DIAGNOSIS AND TREATMENT DEPT. is displayed (ST702). Select a patient in this screen (ST703). After selecting a patient, select OK/CANCEL (ST704). If OK is selected, a communicator screen for the selected patient is displayed (ST705). In the patient selection menu screen, click CLOSE button (ST706) to end the operation.

Referring back to FIG. 3, if an event window for a doctor, a nurse, or another occupation type is clicked (ST127), the event window is displayed (ST128). In the event window, select "CONTINUE/CLOSE" (ST801) as shown in FIG. 10. If CONTINUE is selected, set an event (ST802). After setting the event, select OK/CANCEL (ST803). If OK is selected, a new event screen is displayed (ST804). In the event window, click CLOSE button (ST805) to end the operation.

In addition, the clinical communication device allows the doctor's diagnosis and treatment record on a given date, an order history or a flow sheet (a follow-up sheet) to be invoked, making it possible to invoke and display a clinical examination result or an imaging examination result.

The present invention is not limited to the above-described embodiment, and various modifications can be made thereto. For example, the screen configuration and display items of the clinical communication device are merely illustrative, and various modifications can be made without being limited thereto. In addition, numerical values and medicine names on the display screen are also not limited.

### BRIEF DESCRIPTION OF DRAWINGS

FIG. 1 is a diagram illustrating a schematic configuration of a hospital information system according to an embodiment of the present invention.
FIG. 2 is a diagram illustrating a schematic configuration of a clinical communication device according to an embodiment of the present invention.
FIG. 3 is a flow chart for explaining a procedure for operating a clinical communication device according to an embodiment of the present invention.
FIG. 4 is a flow chart for explaining a procedure for operating the clinical communication device according to the embodiment of the present invention.
FIG. 5 is a flow chart for explaining a procedure for operating the clinical communication device according to the embodiment of the present invention.
FIG. 6 is a flow chart for explaining a procedure for operating the clinical communication device according to the embodiment of the present invention.

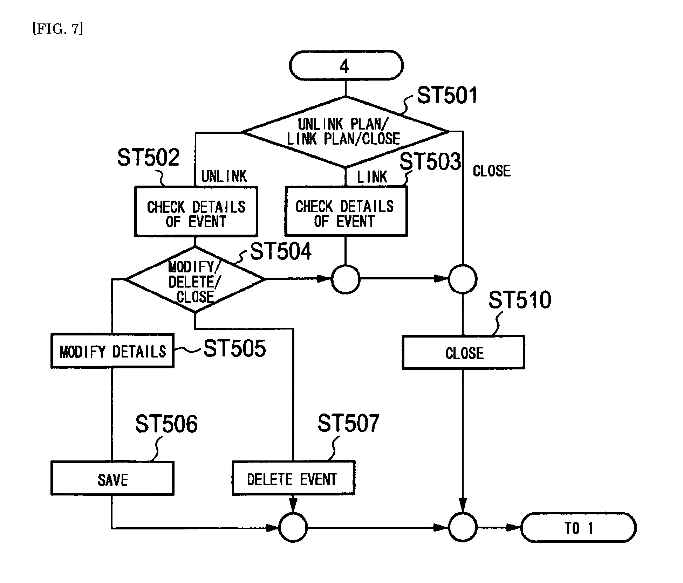

FIG. 7 is a flow chart for explaining a procedure for operating the clinical communication device according to the embodiment of the present invention.
FIG. 8 is a flow chart for explaining a procedure for operating the clinical communication device according to the embodiment of the present invention.
FIG. 9 is a flow chart for explaining a procedure for operating the clinical communication device according to the embodiment of the present invention.
FIG. 10 is a flow chart for explaining a procedure for operating the clinical communication device according to the embodiment of the present invention.
FIG. 11 is a diagram illustrating a display screen when operating the clinical communication device according to the embodiment of the present invention.
FIG. 12 is a diagram illustrating a display screen when operating the clinical communication device according to the embodiment of the present invention.

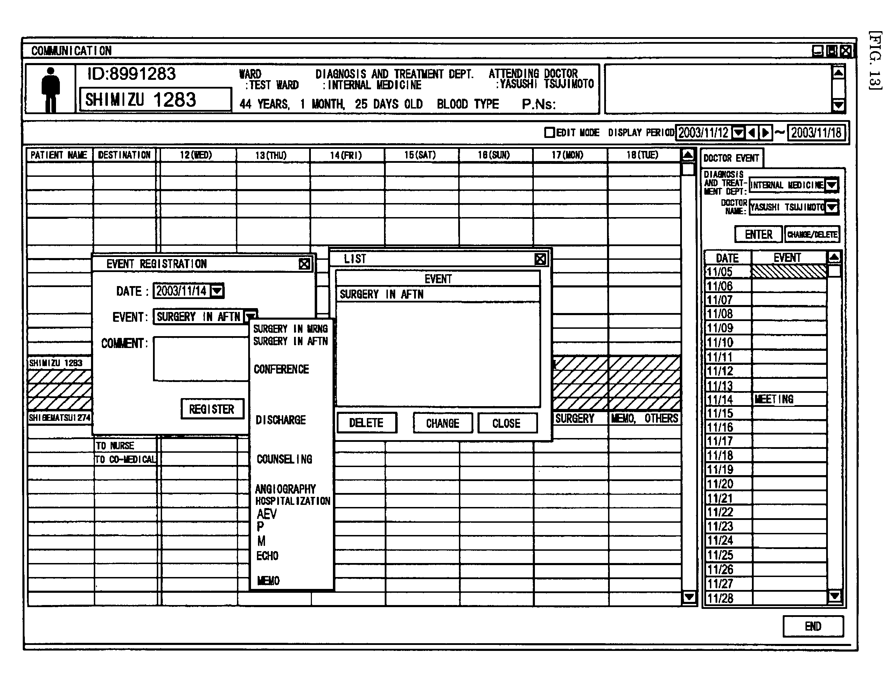

FIG. 13 is a diagram illustrating a display screen when operating the clinical communication device according to the embodiment of the present invention.
FIG. 14 is a diagram illustrating a display screen when operating the clinical communication device according to the embodiment of the present invention.
FIG. 15 is a diagram illustrating a display screen when operating the clinical communication device according to the embodiment of the present invention.

### EXPLANATIONS OF LETTERS OR NUMERALS

- 1,2: clinical communication device
- 3: electronic medical record database
- 4: clinical communicator database
- 5, 6: communication link
- 11: control section
- 12: communication control section
- 13: electronic medical record information extraction section
- 14: clinical communicator information extraction section
- 15: information update section
- 16: display control section
- 17: display

## Claims

1. A clinical communication device comprising:
electronic medical record information extraction means for extracting electronic medical record information from an electronic medical record database, the electronic medical record information including at least clinical pathways, medical record information for patients and follow-up sheets for the patients;
clinical communicator information extraction means for extracting clinical communication information from a clinical communicator database, the clinical communication information including at least event information for the patients, event information for medical care professionals and messages to/from the medical care professionals;
information update means for updating the electronic medical record information and/or the clinical communication information in accordance with an input; and
display control means for displaying the electronic medical record information and the clinical communication information, while linking them with each other.

2. A clinical communication device as claimed in claim 1, wherein the electronic medical record information includes diagnosis information and order information, and the display control means generates a scheduling table containing the event information for the patients and/or the event information for the medical care professionals and displays the diagnosis and treatment records, the order information, the follow-up sheets and/or the clinical pathways from the scheduling table.

3. A clinical communication device as claimed in claim 1 or 2, wherein the information update means sets an importance level to information that is to be exchanged between the medical care professionals.

4. A clinical communication device as claimed in any one of claims 1 to 3, wherein the display control means issues an alert when a scheduled time to implement an event for the patients and/or an event for the medical care professionals has passed.

5. A hospital information system comprising:
an electronic medical record database having stored therein electronic medical record information including at least clinical pathways, medical record information for patients and follow-up sheets for the patients;
a clinical communicator database having stored therein clinical communication information including at least event information for the patients, event information for medical care professionals and messages to/from the medical care professionals; and
a clinical communication device of any one of claims 1 to 4 connected to the electronic medical record database and the clinical communicator database through a communication link.
